# EUROPÄISCHE PATENTANMELDUNG

(11) **EP 4 656 238 A2**
(43) Veröffentlichungstag der Anmeldung: **03.12.2025**
(21) Anmeldenummer: 25200950.1
(22) Anmeldetag: 12.06.2019
(51) Int. Cl.: A61P 3/00

(54) **VERFAHREN ZUR HERSTELLUNG VON ACYLVERKAPPTEN 3-HYDROXYCARBONSÄUREN SOWIE DEREN SALZEN UND ESTERN**

(62) Teilanmeldung aus: 19731649.0
(71) Anmelder: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Strehlke, Ingo Kurt

(57) **Zusammenfassung**

Die Erfindung betrifft ein Verfahren zur Herstellung von gegebenenfalls funktionalisierten acylverkappten (acylblockierten) 3-Hydroxybuttersäuren und deren Salzen und Estern sowie die auf diese Weise erhältlichen Produkte und deren Verwendung.

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von gegebenenfalls funktionalisierten acylverkappten oder acylblockierten 3-Hydroxybuttersäuren sowie deren Salzen und Estern sowie die auf diese Weise erhältlichen bzw. hergestellten Reaktionsprodukte (d. h. gegebenenfalls funktionalisierte acylverkappte oder acylblockierte 3-Hydroxybuttersäuren sowie deren Salze und Ester) und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. gegebenenfalls funktionalisierte acylverkappte oder acylblockierte 3-Hydroxybuttersäuren sowie deren Salze und Ester) umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel (*Functional Food*), *Novel Food,* Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Reaktionsprodukte (d. h. gegebenenfalls funktionalisierte acylverkappte oder acylblockierte 3-Hydroxybuttersäuren sowie deren Salze und Ester) umfassen, sowie deren Anwendungen bzw. Verwendungen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter dem Begriff der Ketokörper fasst man insbesondere die drei Verbindungen Acetoacetat (synonym auch Acetacetat oder 3-Oxobutyrat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoacidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure sowie Acetoacetat als physiologischer Vorläufer der 3-Hydroxybuttesäure demgegenüber ein wirksameres pharmazeutisch-pharmakologisches Zielmolekül dar, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure sowie für Acetoacetat (und damit für die physiologisch durch Reduktion von Acetoacetat erhältliche 3-Hydroxybuttersäure oder dessen Salz).

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen sowie zu Acetoacetat (und damit physiologisch zu 3-Hydroxybuttersäure oder deren Salzen) ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. beta-Hydroxybuttersäure bzw. BHB bzw. 3-BHB) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass gegebenenfalls funktionalisierte acylverkappte oder acylblockierte 3-Hydroxybuttersäuren sowie deren Salze und Ester, insbesondere die Ester der gegebenenfalls funktionalisierten acylverkappten oder acylblockierten 3-Hydroxybuttersäuren, einen effizienten und physiologisch wirksamen bzw. physiologisch kompatiblen Präkursor und/oder Metaboliten für den Ketokörper 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von gegebenenfalls funktionalisierter acylverkappter (acylblockierter) 3-Hydroxybuttersäure (beta-Hydroxybuttersäure, BHB bzw. 3-BHB) oder deren Salzen oder Estern gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung - ein nach dem erfindungsgemäßen Verfahren erhältliches Reaktionsprodukt gemäß dem diesbezüglichen unabhängigen Anspruch (Anspruch 23) bzw. eine gegebenenfalls funktionalisierte acylverkappte (acylblockierte) 3-Hydroxybuttersäure (beta-Hydroxybuttersäure, BHB bzw. 3-BHB) oder deren Salz oder Ester gemäß den diesbezüglichen Ansprüchen (Ansprüche 29 bis 31) bzw. ein diesbezüglich erhältliches Gemisch von mindestens zwei, insbesondere mindestens drei gegebenenfalls funktionalisierten acylverkappten (acylblockierten) 3-Hydroxybuttersäuren (beta-Hydroxybuttersäuren, BHB bzw. 3-BHB) oder deren Salzen oder Estern gemäß den diesbezüglichen Ansprüchen (Ansprüche 32 und 33); weitere, insbesondere besondere und/oder vorteilhafte Ausgestaltungen dieses Erfindungsaspekts sind Gegenstand der diesbezüglichen Unteransprüche.

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 34); weitere, insbesondere besondere und/oder vorteilhafte Ausgestaltungen dieses Erfindungsaspekts sind Gegenstand des diesbezüglichen Unteranspruchs.

Darüber hinaus betrifft die vorliegende Erfindung - gemäß einem **vierten** Aspekt der vorliegenden Erfindung - ein erfindungsgemäßes Reaktionsprodukt bzw. eine erfindungsgemäße gegebenenfalls funktionalisierte acylverkappte (acylblockierte) 3-Hydroxybuttersäure (beta-Hydroxybuttersäure, BHB bzw. 3-BHB) oder deren Salz oder Ester bzw. ein erfindungsgemäßes Gemisch von mindestens zwei, insbesondere mindestens drei gegebenenfalls funktionalisierten acylverkappten (acylblockierten) 3-Hydroxybuttersäuren (beta-Hydroxybuttersäuren, BHB bzw. 3-BHB) oder deren Salzen oder Estern zur prophylaktischen und/oder therapeutischen Behandlung bzw. zur Verwendung bei der prophylaktischen und/oder therapeutischen Behandlung von Erkrankungen des menschlichen oder tierischen Körpers gemäß dem diesbezüglichen unabhängigen Anspruch (Anspruch 36).

Des Weiteren betrifft die vorliegende Erfindung - gemäß einem **fünften** Aspekt der vorliegenden Erfindung - die Verwendung eines erfindungsgemäßen Reaktionsprodukts bzw. einer erfindungsgemäßen gegebenenfalls funktionalisierten acylverkappten (acylblockierten) 3-Hydroxybuttersäure (beta-Hydroxybuttersäure, BHB bzw. 3-BHB) oder deren Salz oder Ester bzw. eines erfindungsgemäßen Gemischs von mindestens zwei, insbesondere mindestens drei gegebenenfalls funktionalisierte acylverkappten (acylblockierten) 3-Hydroxybuttersäuren (beta-Hydroxybuttersäuren, BHB bzw. 3-BHB) oder deren Salzen oder Estern zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers gemäß dem diesbezüglichen unabhängigen Anspruch (Anspruch 37).

Darüber hinaus betrifft die vorliegende Erfindung - gemäß einem **sechsten** Aspekt der vorliegenden Erfindung - die Verwendung eines erfindungsgemäßen Reaktionsprodukts bzw. einer erfindungsgemäßen gegebenenfalls funktionalisierten acylverkappten (acylblockierten) 3-Hydroxybuttersäure (beta-Hydroxybuttersäure, BHB bzw. 3-BHB) oder deren Salz oder Ester bzw. eines erfindungsgemäßen Gemischs von mindestens zwei, insbesondere mindestens drei gegebenenfalls funktionalisierten acylverkappten (acylblockierten) 3-Hydroxybuttersäuren (beta-Hydroxybuttersäuren, BHB bzw. 3-BHB) oder deren Salzen oder Estern gemäß dem diesbezüglichen unabhängigen Anspruch (Anspruch 38).

Des Weiteren betrifft die vorliegende Erfindung - gemäß einem **siebten** Aspekt der vorliegenden Erfindung - ein Nahrungsmittel und/oder Lebensmittelerzeugnis gemäß dem diesbezüglichen unabhängigen Anspruch (Anspruch 39); weitere, insbesondere besondere und/oder vorteilhafte Ausgestaltungen des erfindungsgemäßen Nahrungsmittel- und/oder Lebensmittelerzeugnisses sind Gegenstand des diesbezüglichen Unteranspruchs.

Schließlich betrifft die vorliegende Erfindung - gemäß einem **achten** Aspekt der vorliegenden Erfindung - die Verwendung eines erfindungsgemäßen Reaktionsprodukts bzw. einer erfindungsgemäßen gegebenenfalls funktionalisierten acylverkappten (acylblockierten) 3-Hydroxybuttersäure (beta-Hydroxybuttersäure, BHB bzw. 3-BHB) oder deren Salz oder Ester bzw. eines erfindungsgemäßen Gemischs von mindestens zwei, insbesondere mindestens drei gegebenenfalls funktionalisierten acylverkappten (acylblockierten) 3-Hydroxybuttersäuren (beta-Hydroxybuttersäuren, BHB bzw. 3-BHB) oder deren Salzen oder Estern in einem Nahrungsmittel- und/oder Lebensmittelerzeugnis gemäß dem diesbezüglichen unabhängigen Anspruch (Anspruch 41); weitere, insbesondere besondere und/oder vorteilhafte Ausgestaltungen der erfindungsgemäßen Verwendung sind Gegenstand des diesbezüglichen Verwendungsunteranspruchs.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist ein Verfahren zur Herstellung von acylverkappter (= acylblockierter) 3-Hydroxybuttersäure (= beta-Hydroxybuttersäure, BHB bzw. 3-BHB) oder deren Salz oder Ester,
wobei mindestens eine Verbindung der allgemeinen Formel (I)

   CH₃ - CH(OH) - CH₂ - C(O)OR¹ (I)
wobei in der allgemeinen Formel (I) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt,
mit mindestens einer Verbindung der allgemeinen Formel (II)

   CH₃ - C(O) - CH₂ - C(O)OR² (II)
wobei in der in der allgemeinen Formel (II) der Rest R² ein C₁-C₄-Alkyl, insbesondere Methyl oder Ethyl, bevorzugt Ethyl, darstellt,
umgesetzt wird,
so dass als Reaktionsprodukt mindestens eine acylverkappte (acylblockierte) 3-Hydroxybuttersäure oder deren Salz oder Ester der allgemeinen Formel (III)

   CH₃ - CH(OR³) - CH₂ - C(O)OR¹ (III)
wobei in der allgemeinen Formel (III) der Rest R¹ die zuvor angegebene Bedeutung hat und der Rest R³ einen Rest CH₃ - C(O) - CH₂ - C(O) - darstellt,
erhalten wird.

Nach dem erfindungsgemäßen Verfahren resultiert also eine in 3-Position (= Hydroxylgruppen-Position) mit einer Acylgruppe verkappte bzw. blockierte 3-Hydroxybuttersäure bzw. deren Salz oder Ester. Bei einer Acylgruppe handelt es sich um eine funktionelle Gruppe in der organischen Chemie mit der allgemeinen Struktur R - (C = O) -, wobei der Rest R einen Organyl-Rest (Alkyl-, Aryl- oder eine heteroaromatische Gruppe etc.) oder ein Wasserstoffatom darstellt. Die Acylgruppe leitet sich formal von Carbonsäuren, Aldehyden und Carbonsäurechloriden ab, in denen eine OH-Gruppe, ein Wasserstoffatom bzw. ein Chlorid durch einen Rest R substituiert wurde. Eine Acylierung bezeichnet die Einführung einer solchen Acylgruppe.

Für den Fall, dass (wie im Fall der Erfindung) die Acylierung an einer Hydroxylgruppe (OH-Gruppe) stattfindet (nämlich an der in 3-Position der 3-Hydroxybuttersäure befindlichen OH-Gruppe), wird insgesamt eine Acyloxygruppe gebildet, welche die allgemeine Struktur R - (C = O) - O - hat.

Erfindungsgemäß ist eine acylverkappte (= acylblockierte) 3-Hydroxybuttersäure also eine in 3-Position acylierte Buttersäure bzw. eine in 3-Position acyloxylierte Butansäure.

Wie zuvor ausgeführt, hat die Anmelderin nämlich vollkommen überraschend herausgefunden, dass die auf diese Weise hergestellten acylverkappten (= acylblockierten) 3-Hydroxybuttersäuren oder deren Salze oder Ester (welche gegebenenfalls zusätzlich auch noch funktionalisiert sein können, wie nachfolgend noch im Detail beschrieben) effiziente, da physiologisch verträgliche Präkursoren und/oder Metabolite der freien 3-Hydroxybuttersäure bzw. deren Salzen oder Ester darstellen, welche pharmazeutisch bzw. klinisch auch in größeren Mengen zum Einsatz kommen können, da sie physiologisch kompatibel sind.

Die vorgenannten, gegebenenfalls funktionalisierten acylverkappten (= acylblockierten) 3-Hydroxybuttersäuren oder deren Salze oder Ester, welche durch das erfindungsgemäße Herstellungsverfahren erstmals in effizienter Weise zugänglich sind, stellen also eine physiologisch und pharmakologisch relevante Alternative zu der freien 3-Hydroxybuttersäure bzw. deren Salzen oder Ester dar.

Die Herstellung derartiger Verbindungen mittels herkömmlicher organischer Synthese ist komplex und aufwendig, da 3-Hydroxybuttersäure verstärkt zur Polymerisation und anderen unerwünschten Nebenreaktionen (z. B. Wasserabspaltung, Zersetzung etc.) neigt. Im Rahmen der vorliegenden Erfindung konnte erstmals ein effizient arbeitendes Herstellungsverfahren bereitgestellt werden, mit welchem sich gegebenenfalls funktionalisierte acylverkappte (acylblockierte) 3-Hydroxybuttersäuren sowie deren Salze und Ester ohne unerwünschte Nebenreaktionen herstellen lassen, insbesondere einstufig.

Das erfindungsgemäße Verfahren ermöglicht somit erstmalig die Bereitstellung untoxischer, gegebenenfalls funktionalisierter acylverkappter (acylblockierter) 3-Hydroxybuttersäuren sowie deren Salzen und Estern aus an sich bekannten, kommerziell verfügbaren und vor allem physiologisch unbedenklichen Komponenten bzw. Edukten (Ausgangsverbindungen). Die resultierenden gegebenenfalls funktionalisierten acylverkappten 3-Hydroxybuttersäuren sowie deren Salze und Ester können physiologisch, insbesondere im Magen und/oder im Darm, aufgespalten werden und das Zielmolekül "3-Hydroxybuttersäure" bzw. deren Salze (und daneben auch Acetoacetat, welches physiologisch wiederum weiter zu 3-Hydroxybuttersäure umgesetzt bzw. reduziert werden kann) als Wirkstoff bzw. Wirkkomponente freisetzen bzw. generieren.

Darüber hinaus weisen die vorgenannten, gegebenenfalls funktionalisierten acylverkappten (acylblockierten) 3-Hydroxybuttersäuren sowie deren Salze und Ester auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Weiterhin zeigen Untersuchungen der Anmelderin, dass die erfindungsgemäßen gegebenenfalls funktionalisierten acylverkappte (acylblockierte) 3-Hydroxybuttersäure bzw. deren Salze oder Ester nicht nur selbst effiziente Präkursoren bzw. Metabolite der freien Hydroxybuttersäure bzw. deren Salze darstellen, sondern auch als Edukte für die Synthese weiterer Präkursoren bzw. Metabolite der freien Hydroxybuttersäure bzw. deren Salze (z. B. Glyceride) verwendet werden können.

Gleichermaßen ermöglicht es das erfindungsgemäße Herstellungsverfahren, die acylverkappten (acylblockierten) 3-Hydroxybuttersäuren sowie deren Salze und Ester frei von toxischen Verunreinigungen bereitzustellen.

Bei der physiologischen Spaltung im Magen und/oder Darm wird die gegebenenfalls funktionalisierte acylverkappte (acylblockierte) 3-Hydroxybuttersäure in die Ketoverbindungen 3-Hydroxybuttersäure und 3-Oxobutyrat (Acetoacetat bzw. Acetacetat), welches vom Körper weiter zu 3-Hydroxybutyrat reduziert werden kann, gespalten. Durch die Anwesenheit von sowohl 3-Oxobutyratresten und 3-Hydroxybutyratresten bzw. 3-Hydroxybuttersäure liegt eine unterschiedlich schnelle Verfügbarkeit bzw. Freisetzung des Wirkstoffs 3-Hydroxybuttersäure vor. Das erfindungsgemäße Reaktionsprodukt weist folglich einen Retard-Effekt auf. Insgesamt weist die erfindungsgemäße gegebenenfalls funktionalisierte acylverkappte (acylblockierte) 3-Hydroxybuttersäure somit zwei Ketokörper mit unterschiedlich schnellem Abbau auf.

Weiterhin kann durch eine gezielte Steuerung der Reaktionsbedingungen, insbesondere der Eduktmengen und/oder Verhältnisse, eine doppelte Verkappung (d. h. Bildung eines 3-BHB-Dimers, welches anschließend durch eine erfindungsgemäße Ketoverbindung verkappt wird) erfolgen, wodurch eine wiederum längerfristige Verfügbarkeit des Wirkstoffs 3-Hydroxybuttersäure ermöglicht wird.

Darüber hinaus kann bei entsprechender Auswahl der Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer anzureichern bzw. zu erhalten, um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer anzureichern bzw. zu erhalten.

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare Edukte und ermöglicht darüber hinaus eine relativ einfache Verfahrensführung auch bei großtechnischer Umsetzung. Die eingesetzten Edukte sind weiterhin selbst physiologisch kompatibel und sogar pharmazeutisch wirksam, sodass gegebenenfalls noch vorhandene Edukte im Reaktionsprodukt verbleiben können und keine bzw. kaum Aufreinigungsverfahrensschritte notwendig sind.

Grundsätzlich ist es aber möglich und kann unter bestimmten Voraussetzungen, insbesondere im Hinblick auf die organoleptischen Eigenschaften, zweckdienlich sein, die Edukte aus dem Reaktionsprodukt zu entfernen.

Im Gegensatz zu herkömmlichen Herstellungsverfahren des Standes der Technik kommt das erfindungsgemäße Herstellungsverfahren ohne komplexe Edukte aus und verläuft nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird.

Darüber hinaus ist das erfindungsgemäßen Verfahren einfach und wirtschaftlich. Insbesondere wird üblicherweise das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Das erfindungsgemäße Herstellungsverfahren von acylverkappter (acylblockierter) 3-Hydroxybuttersäure wird im nachfolgenden allgemeinen Reaktionsschema veranschaulicht (wobei die Reste R¹ und R² die zuvor angegebene Bedeutung haben und "Kat" einen Katalysator bezeichnet):

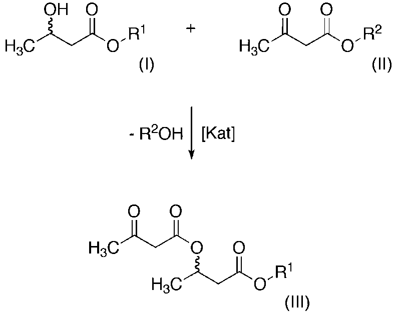

Gemäß einer besonderen Ausführungsform der vorliegenden Erfindung kann die Verbindung der allgemeinen Formel (I) entweder in racemischer Form oder in Form des (R)-Enantiomers eingesetzt werden. Die (R)-Konfiguration bezieht sich auf das chirale Kohlenstoffatom in 3-Position der Verbindung der allgemeinen Formel (I).

Gemäß einer bevorzugten Ausführungsform kann die Verbindung der allgemeinen Formel (I) einen Ester sein (d. h. in der obigen allgemeinen Formel (I) stellt der Rest R¹ ein C₁-C₄-Alkyl dar bzw. stellt der Rest R¹ keinen Wasserstoff dar).

Insbesondere kann es im Rahmen des erfindungsgemäßen Herstellungsverfahrens bevorzugt sein, wenn in der obigen allgemeinen Formel (I) der Rest R¹ Ethyl darstellt. Mit anderen Worten kann als Verbindung der allgemeinen Formel (I) 3-Hydroxybuttersäureethylester (Ethyl-3-hydroxybutyrat) der Formel CH₃ -CH(OH) - CH₂ - C(O)OC₂H₅ eingesetzt werden.

Darüber hinaus kann es gemäß des erfindungsgemäßen Herstellungsverfahren bevorzugt sein, wenn in der obigen allgemeinen Formel (II) der Rest R² Ethyl darstellen. Mit anderen Worten wird bei dieser Ausführungsform als Verbindung der allgemeinen Formel (II) 3-Oxobuttersäureethylester (Ethyl-3-oxobutyrat) der Formel CH₃ - C(O) - CH₂ - C(O)OC₂H₅ eingesetzt.

Gemäß einer besonderen Ausführungsform der vorliegenden Erfindung betrifft die vorliegende Erfindung ein Verfahren zur Herstellung von acylverkappter (acylblockierter) 3-Hydroxybuttersäure (beta-Hydroxybuttersäure, BHB bzw. 3-BHB) oder deren Salz oder Ester, insbesondere wie zuvor definiert,
wobei mindestens eine Verbindung der Formel (la)

   CH₃ - CH(OH) - CH₂ - C(O)OC₂H₅ (la)
mit mindestens einer Verbindung der Formel (Ila)

   CH₃ - C(O) - CH₂ - C(O)OC₂H₅ (IIa)
umgesetzt wird,
so dass als Reaktionsprodukt mindestens eine acylverkappte (acylblockierte) 3-Hydroxybuttersäure oder deren Salz oder Ester der Formel (IIIa)

   CH₃ - CH[O - C(O) - CH₂ - C(O) - CH₃] - CH₂ - C(O)OC₂H₅ (IIIa)
erhalten wird.

Die erfindungsgemäß besonders bevorzugte Ausführungsform, wonach die Verbindungen der allgemeinen Formel (I) und (II) Ethylester sind, wird durch das folgende Reaktionsschema veranschaulicht:

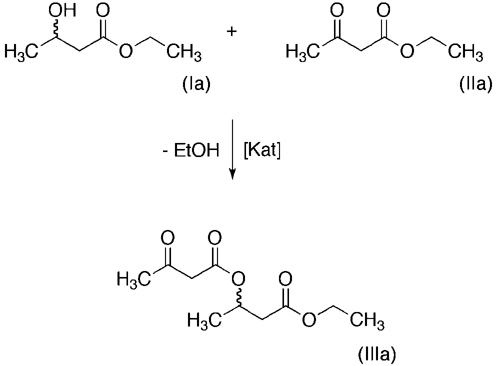

Diese besondere Ausführungsform, wonach die Verbindungen der allgemeinen Formel (I) und (II) Ethylester sind, ermöglicht eine besonders effiziente Verfahrensführung und hohe Ausbeuten mit minimierter bzw. unterdrückter Nebenproduktbildung. Zudem sind sowohl der 3-Hydroxybuttersäureethylester als auch der 3-Oxobuttersäureethylester in größeren Mengen kommerziell verfügbar und zudem ökonomisch effizient umsetzbar. Insbesondere ist der 3-Hydroxybuttersäureethylester ökonomisch effizienter als die freie Säure (d. h. 3-Hydroxybuttersäure). Darüber hinaus können die Ausgangsverbindungen (d. h. der 3-Hydroxybuttersäureethylester und der 3-Oxobuttersäureethylester) großtechnisch z. B. durch Claisen-Kondensation von Ethylacetat gewonnen werden.

Insbesondere wird bei dem erfindungsgemäßen Verfahren die Umsetzung in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenprodukte.

Gemäß einer besonderen Ausführungsform der vorliegenden Erfindung kann die Umsetzung in Gegenwart eines Katalysators, insbesondere eines Enzyms und/oder eines metallhaltigen und/oder metallbasierten, sauren oder basischen Katalysators, vorzugsweise in Gegenwart eines Enzyms, durchgeführt werden. Bei dieser besonderen Ausführungsform ist es bevorzugt, wenn der Katalysator nach der Umsetzung rezykliert wird.

Wie zuvor ausgeführt, kann gemäß einer besonderen Ausführungsform des erfindungsgemäßen Herstellungsverfahrens die Umsetzung in Gegenwart eines Enzyms als Katalysator durchgeführt werden.

Dabei kann das Enzym insbesondere ausgewählt sein aus Synthetasen (Ligasen), Katalasen, Esterasen, Lipasen und deren Kombinationen. Erfindungsgemäß werden als Synthetasen (Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasserstoff umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstande sind (Lipolyse).

Im Rahmen der vorliegenden Erfindung kann sich das als Katalysator eingesetzte Enzym insbesondere ableiten von *Candida antarctica, Mucor miehei* (*Rhizomucor miehei*), *Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus.*

Gemäß einer besonderen Ausführungsform kann das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Wie zuvor im Zusammenhang mit der Verwendung eines Katalysators im Allgemeinen dargelegt, ist es bevorzugt, im Fall der Verwendung eines Enzyms als Katalysator das Enzym nach der Umsetzung zu rezyklieren.

Sofern die Umsetzung im Rahmen des erfindungsgemäßen Herstellungsverfahrens in Gegenwart eines Enzyms als Katalysator durchgeführt wird, ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen wird.

Wenn gemäß einer besonderen Ausführungsform der vorliegenden Erfindung die Umsetzung in Gegenwart eines Enzyms als Katalysator durchgeführt wird, kann auch der Druckbereich in weiten Bereichen variieren. Insbesondere kann bei Umsetzung in Gegenwart eines Enzyms als Katalysator die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Gemäß einer alternativen Ausführungsform der vorliegenden Erfindung kann die Umsetzung in Gegenwart eines metallhaltigen und/oder metallbasierten, sauren oder basischen Katalysators durchgeführt werden.

Gemäß dieser alternativen Ausführungsform der vorliegenden Erfindung, wonach die Umsetzung in Gegenwart eines metallhaltigen und/oder metallbasierten, sauren oder basischen Katalysators durchgeführt wird, kann der Katalysator insbesondere ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere ein Alkali- oder Erdalkalialkoholat eingesetzt werden.

Insbesondere ist es auch bei dieser Ausführungsform bevorzugt, wenn der Katalysator auf Basis des metallhaltigen und/oder metallbasierten, sauren oder basischen Katalysators nach der Umsetzung rezykliert wird.

Wenn gemäß dieser besonderen Ausführungsform der vorliegenden Erfindung die Umsetzung in Gegenwart eines metallhaltigen und/oder metallbasierten, sauren oder basischen Katalysators erfolgt, können die Temperaturen in weiten Bereichen variiert werden. Insbesondere kann die Umsetzung in Gegenwart eines metallhaltigen und/oder metallbasierten, sauren oder basischen Katalysators bei Temperaturen im Bereich von 20 °C bis 150 °C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt werden.

Weiterhin kann auch bei dieser Ausführungsform der Katalysator (d. h. der metallhaltige und/oder metallbasierte, saure oder basische Katalysator) in weiten Mengenbereichen variiert werden: So kann der Katalysator auf Basis eines metallhaltigen und/oder metallbasierten, sauren oder basischen Katalysators in Mengen, bezogen auf die Gesamtmenge der Ausgangsverbindungen (I) und (II), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt werden. Dennoch ist es anwendungsbezogen oder einzelfallbedingt möglich, von den genannten Mengen abzuweichen, ohne dass der Rahmen der vorliegenden Erfindung verlassen ist.

Wenn gemäß dieser besonderen Ausführungsform der vorliegenden Erfindung die Umsetzung in Gegenwart eines metallhaltigen und/oder metallbasierten, sauren oder basischen Katalysators erfolgt, kann der Druckbereich gleichermaßen in weiten Bereichen variieren: Insbesondere kann die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Was die Menge an Edukten bzw. Ausgangsverbindungen anbelangt, so kann auch diese in weiten Bereichen variiert werden.

Unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (II), bezogen auf die Verbindung der allgemeinen Formel (I), in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (II) und die Verbindung der allgemeinen Formel (I) in einem Molverhältnis von Verbindung der allgemeinen Formel (II) / Verbindung der allgemeinen Formel (I) in einem Bereich von 1,1 : 1 bis 10 : 1, bevorzugt in einem Bereich von 1,5 : 1 bis 9 : 1, insbesondere in einem Bereich von 2 : 1 bis 8 : 1, vorzugsweise in einem Bereich von 3 : 1 bis 6 : 1, eingesetzt werden. Auf diese Weise wird einer Nebenproduktbildung, insbesondere der Bildung von dimerer 3-Hydroxybuttersäure und deren acylverkappten Derivaten, in effizienter Weise entgegengewirkt.

Im Rahmen des erfindungsgemäßen Herstellungsverfahrens wird bei der Umsetzung der mindestens einen Verbindung der allgemeinen Formel (I) mit mindestens einer Verbindung der allgemeinen Formel (II) gleichzeitig eine Verbindung gemäß der allgemeinen Formel (IV)

R² - OH (IV)

wobei der Rest R² die zuvor angegebene Bedeutung hat, gebildet. Daher kann es erfindungsgemäß insbesondere vorgesehen sein, dass die Verbindung gemäß der allgemeinen Formel (IV) der Umsetzung insbesondere kontinuierlich entzogen wird, insbesondere mittels vorzugsweise kontinuierlicher destillativer Entfernung. Auf diese Weise wird das Reaktionsgleichgewicht in effizienter Weise auf die Seite der Reaktionsprodukte (d. h. der acylverkappten (acylblockierten) 3-Hydroxybuttersäure oder deren Salz oder Ester der allgemeinen Formel (III)) verlagert. Auch wird auf diese Weise die Bildung von Nebenprodukten minimiert bzw. verhindert.

Im Anschluss an die Umsetzung kann das erhaltene Reaktionsprodukt weiteren üblichen bzw. an sich bekannten Aufreinigungs- bzw. Aufarbeitungsschritten unterzogen werden.

In diesem Zusammenhang kann sich der Umsetzung der mindestens einen Verbindung der allgemeinen Formel (I) mit mindestens einer Verbindung der allgemeinen Formel (II) eine Aufreinigung anschließen, insbesondere mittels Destillation und/oder Chromatographie, vorzugsweise mittels Destillation.

Auch können nicht umgesetzte bzw. noch vorhandene Edukte und Reaktionsnebenprodukte, insbesondere Verbindungen gemäß der allgemeinen Formel (IV), abgetrennt werden, insbesondere abdestilliert werden.

Im Rahmen der vorliegenden Erfindung können insbesondere gegebenenfalls noch vorhandene Edukte, insbesondere Edukte der allgemeinen Formeln (I) und (II), nach deren Abtrennung rezykliert werden.

Gemäß einer besonderen Ausführungsform des erfindungsgemäßen Herstellungsverfahrens kann insbesondere derart vorgegangen werden, dass nach erfolgter Umsetzung das Reaktionsprodukt (III) an dessen Rest R¹ zumindest teilweise, vorzugsweise vollständig, funktionalisiert wird, vorzugsweise durch Veresterung oder Umesterung.

Insbesondere kann sich im Rahmen der vorliegenden Erfindung der Umsetzung eine teilweise, insbesondere vollständige, Funktionalisierung des Reaktionsprodukts (III) an dessen Rest R¹ anschließen, vorzugsweise durch Veresterung oder Umesterung.

Im Rahmen der vorliegenden Erfindung kann unter Funktionalisierung der Austausch bzw. das Einführen bestimmter Seitengruppen bzw. funktioneller Gruppen verstanden werden. Eine Veresterung findet statt, wenn der Rest R¹ ein Wasserstoff darstellt und somit das Reaktionsprodukt (III) in Form einer Carbonsäure vorliegt. Diese Carbonsäure wird im Rahmen der Veresterung mit einem Alkohol umgesetzt, sodass ein Ester unter Wasserabspaltung gebildet wird. Wenn jedoch der Rest R¹ im Reaktionsprodukt (III) ein C₁-C₄-Alkyl darstellt, findet eine Umesterung statt. Bei einer Umesterung wird ein Ester in einen anderen Überführt. Dabei wird der Alkoholrest eines Ester (d. h. in diesem Fall enthaltend den C₁-C₄-Alkylrest) durch einen anderen Alkoholrest ersetzt.

In diesem Zusammenhang ist es insbesondere bevorzugt, wenn das Reaktionsprodukt (III) mit mindestens einem Fettalkohol (V), vorzugsweise ausgewählt aus C₆-C₃₀-Fettalkoholen, bevorzugt C₁₀-C₃₀-Fettalkoholen, insbesondere C₁₀-C₂₄-Fettalkoholen, umgesetzt wird.

Gemäß einer bevorzugten Ausführungsform der vorliegenden Erfindung, entspricht der Fettalkohol (V) der allgemeinen Formel (V')

R⁴ - OH (V')

wobei der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt, insbesondere wobei die Hydroxylfunktion (OH-Funktion) primär und/oder endständig ist.

Bei dieser Ausführungsform des erfindungsgemäßen Verfahrens ist es insbesondere bevorzugt, wenn der Rest R⁴ einen linearen, gesättigten oder ein- oder mehrfach ungesättigten aliphatischen C₁₀-C₂₄-Alkylrest darstellt, insbesondere wobei die Hydroxylfunktion (OH-Funktion) primär und/oder endständig ist.

Insbesondere ist es bevorzugt, wenn der Rest R⁴ einen 1-Decanylrest, einen 1-Dodecanylrest (Laurylrest), einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis*-9-Hexadecen-1-ylrest (Palmitoleylrest), einen *cis*-9-Octadecen-1-ylrest (Oleylrest), einen *trans*-9-Octadecen-1-ylrest (Elaidylrest), einen *cis*-11-Octadecen-1-ylrest, einen *cis*,*cis*-9,12-Octa-decadien-1-ylrest (Linoleylrest) oder einen 6,9,12-Octadecatrien-1-ylrest (γ-Linolenylrest), bevorzugt einen *cis*-9-Octadecen-1-ylrest (Oleylrest), darstellt.

Gemäß einer besonderen Ausführungsform des erfindungsgemäßen Verfahrens, ist es bevorzugt, wenn der Fettalkohol (V) ausgewählt ist aus linearen oder verzweigten, gesättigten oder ein- oder mehrfach ungesättigten aliphatischen C₆-C₃₀-Fettalkoholen, vorzugsweise C₁₀-C₃₀-Fettalkoholen, insbesondere C₁₀-C₂₄-Fettalkoholen, bevorzugt mit primärer und/oder endständiger Hydroxylfunktion (OH-Funktion).

Insbesondere kann der im Rahmen des erfindungsgemäßen Verfahrens einsetzbare Fettalkohol (V) ausgewählt sein aus linearen, gesättigten oder ein- oder mehrfach ungesättigten, aliphatischen einwertigen und bevorzugt primären C₆-C₃₀-Fettalkoholen, vorzugsweise linearen, gesättigten oder ein- oder mehrfach ungesättigten, aliphatischen einwertigen und bevorzugt primären C₁₀-C₃₀-Fettalkoholen, insbesondere linearen, gesättigten oder ein- oder mehrfach ungesättigten, aliphatischen einwertigen und bevorzugt primären C₁₀-C₂₄-Fettalkoholen.

Gemäß einer besonderen Ausführungsform des erfindungsgemäßen Verfahrens kann der Fettalkohol (V) ausgewählt sein aus der Gruppe von 1-Decanol, 1-Dodecanol (Laurylalkohol), 1-Tetradecanol (Myristylalkohol), 1-Hexadecanol (Cetylalkohol), 1-Heptadecanol (Margarylalkohol), 1-Octadecanol (Stearylalkohol), 1-Eicosanol (Arachidylalkohol), 1-Docosanol (Behenylalkohol), 1-Tetracosanol (Ligocerylalkohol), 1-Hexacosanol (Cerylalkohol), 1-Octacosanol (Montanylalkohol), 1-Tricontanol (Melissylalkohol), *cis*-9-Hexadecen-1-ol (Palmitoleylalkohol), *cis*-9-Octadecen-1-ol (Oleylalkohol), *trans*-9-Octadecen-1-ol (Elaidylalkohol), *cis*-11-Octadecen-1-ol, *cis,cis*-9,12-Octadecadien-1-ol (Linoleylalkohol), 6,9,12-Octadecatrien-1-ol (γ-Linolenylalkohol), und deren Mischungen, bevorzugt *cis*-9-Octadecen-1-ol (Oleylalkohol).

Die vorgenannten Fettalkohole (V) sind kommerziell verfügbare chemische Erzeugnisse bzw. ohne Weiteres anderweitig zugänglich.

Im Rahmen der besonderen Ausführungsform der vorliegenden Erfindung, wonach sich der Umsetzung eine teilweise, insbesondere vollständige Funktionalisierung des Reaktionsprodukts (III) an dessen Rest R¹ anschließt, ist es insbesondere bevorzugt, wenn die Funktionalisierung in Abwesenheit von Lösemitteln und/oder ohne jedwedes Lösemittel durchgeführt wird. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Insbesondere ist es gemäß dieser besonderen Ausführungsform bevorzugt, wenn die Funktionalisierung in Gegenwart eines Katalysators, insbesondere eines Enzyms und/oder eines metallhaltigen und/oder metallbasierten, sauren oder basischen Katalysators, vorzugsweise in Gegenwart eines Enzyms, durchgeführt wird. Bei dieser besonderen Ausführungsform ist es bevorzugt, wenn der Katalysator nach der Funktionalisierung rezykliert wird.

Gemäß einer bevorzugten Ausführungsform der Erfindung wird die Funktionalisierung in Gegenwart eines Enzyms als Katalysator durchgeführt.

In diesem Zusammenhang kann das Enzym ausgewählt werden aus Synthetasen (Ligasen), Katalasen, Esterasen, Lipasen und deren Kombinationen.

Im Rahmen der vorliegenden Erfindung kann sich das als Katalysator eingesetzte Enzym insbesondere ableiten von *Candida antarctica, Mucor miehei* (*Rhizomucor miehei*), *Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus.*

Gemäß einer besonderen Ausführungsform kann das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Wie zuvor im Zusammenhang mit der Verwendung eines Katalysators im Allgemeinen dargelegt, ist es bevorzugt, das Enzym nach der Funktionalisierung zu rezyklieren.

Im Rahmen der vorliegenden Erfindung wird die Funktionalisierung in Gegenwart eines Enzyms als Katalysator bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt.

Sofern die Funktionalisierung im Rahmen des erfindungsgemäßen Herstellungsverfahrens in Gegenwart eines Enzyms als Katalysator durchgeführt wird, ist es bevorzugt, wenn das Enzym in Mengen, bezogen auf die Gesamtmenge der Verbindungen (III) und (V), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt wird.

Wenn gemäß einer besonderen Ausführungsform der vorliegenden Erfindung die Funktionalisierung in Gegenwart eines Enzyms als Katalysator durchgeführt wird, kann auch der Druckbereich in weiten Bereichen variieren. Insbesondere kann bei Funktionalisierung in Gegenwart eines Enzyms als Katalysator die Funktionalisierung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Gemäß einer alternativen Ausführungsform der vorliegenden Erfindung kann die Funktionalisierung in Gegenwart eines metallhaltigen und/oder metallbasierten, sauren oder basischen Katalysators durchgeführt werden.

Gemäß dieser alternativen Ausführungsform der vorliegenden Erfindung kann der Katalysator im Rahmen der Funktionalisierung ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Insbesondere kann als Katalysator ein Alkali- oder Erdalkalialkoholat eingesetzt werden.

In diesem Zusammenhang ist es insbesondere bevorzugt, wenn der Katalysator nach der Funktionalisierung rezykliert wird.

Im Rahmen der vorliegenden Erfindung ist es bevorzugt, wenn die Funktionalisierung in Gegenwart eines metallhaltigen und/oder metallbasierten, sauren oder basischen Katalysators bei Temperaturen im Bereich von 20 °C bis 150 °C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt wird.

Sofern die Funktionalisierung im Rahmen des erfindungsgemäßen Herstellungsverfahrens in Gegenwart eines metallhaltigen und/oder metallbasierten, sauren oder basischen Katalysators durchgeführt wird, ist es bevorzugt, wenn der Katalysator in Mengen, bezogen auf die Gesamtmenge der Verbindungen (III) und (V), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt wird.

Wenn gemäß einer besonderen Ausführungsform der vorliegenden Erfindung die Funktionalisierung in Gegenwart eines metallhaltigen und/oder metallbasierten, sauren oder basischen Katalysators durchgeführt wird, kann auch der Druckbereich in weiten Bereichen variieren. Insbesondere kann bei Funktionalisierung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Im Rahmen der erfindungsgemäß bevorzugten Ausführungsform, wonach sich der Umsetzung eine teilweise, insbesondere vollständige Funktionalisierung des Reaktionsprodukts (III) an dessen Rest R¹ anschließt, wird insbesondere bei der Funktionalisierung gleichzeitig eine Verbindung gemäß der allgemeinen Formel (VI)

R¹ - OH (VI)

gebildet, wobei in der allgemeinen Formel (VI) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt.

In diesem Zusammenhang ist es insbesondere bevorzugt, wenn die Verbindung gemäß der allgemeinen Formel (VI) der Funktionalisierung entzogen wird, insbesondere kontinuierlich entzogen wird, insbesondere mittels vorzugsweise kontinuierlicher destillativer Entfernung. Auf diese Weise wird das Reaktionsgleichgewicht in effizienter Weise auf die Seite der Reaktionsprodukte (d. h. Funktionalisierungsprodukte) verlagert. Auch wird auf diese Weise die Bildung von Nebenprodukten minimiert bzw. verhindert.

Eine erfindungsgemäß besonders bevorzugte Vorgehensweise, welche eine Funktionalisierung des Reaktionsprodukts (III) an dessen Rest R¹ im Anschluss an die Umsetzung vorsieht, wird durch das nachfolgende Reaktions- bzw. Syntheseschema mit dem Ethylester der acylverkappten (acylblockierten) 3-Hydroxybuttersäure veranschaulicht (wobei der Rest R⁴ die zuvor angegebene Bedeutung hat):

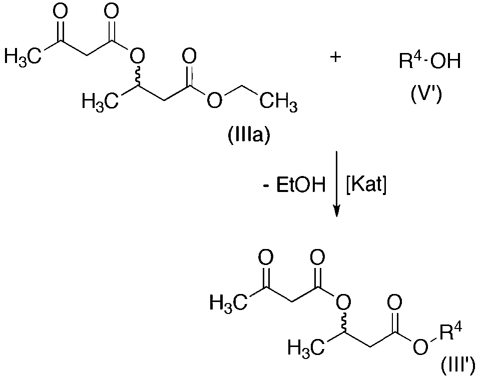

Gemäß dem erfindungsgemäßen Verfahren werden als Reaktionsprodukt ein oder mehrere gegebenenfalls funktionalisierte acylverkappte (acylblockierte) 3-Hydroxybuttersäuren und/oder deren Salze und/oder Ester der allgemeinen Formel (III')

CH₃ - CH(OR³) - CH₂ - C(O)OR⁵ (III')

gebildet,
wobei in der allgemeinen Formel (III') der Rest R³ einen Rest CH₃- C(O) - CH₂ - C(O) - darstellt und der Rest R⁵ einen Rest R¹, wie zuvor definiert, oder einen Rest R⁴, wie zuvor definiert, darstellt.

Gemäß einer besonderen Ausführungsform der vorliegenden Erfindung werden als Reaktionsprodukt ein oder mehrere acylverkappte (acylblockierte) 3-Hydroxybuttersäuren und/oder deren Salze und/oder Ester der allgemeinen Formel (III)

CH₃ - CH(OR³) - CH₂ - C(O)OR¹ (III)

gebildet,
wobei in der allgemeinen Formel (III) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt und der Rest R³ einen Rest CH₃ - C(O) - CH₂ - C(O) - darstellt.

Gemäß einer wiederum besonderen Ausführungsform der vorliegenden Erfindung werden im Rahmen des erfindungsgemäßen Herstellungsverfahrens als Reaktionsprodukt ein oder mehrere funktionalisierte acylverkappte (acylblockierte) 3-Hydroxybuttersäuren und/oder deren Salze und/oder Ester der allgemeinen Formel (III")

CH₃ - CH(OR³) - CH₂ - C(O)OR⁴ (III")

gebildet,
wobei in der allgemeinen Formel (III") der Rest R³ einen Rest CH₃- C(O) - CH₂ - C(O) - darstellt und der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt.

Weiterer Gegenstand - gemäß einem **zweiten** Aspekt der vorliegenden Erfindung - ist das nach dem erfindungsgemäßen Verfahren erhältliche Reaktionsprodukt (d. h. ein (chemisches) Produkt oder Produktgemisch).

Insbesondere ist Gegenstand der vorliegenden Erfindung ein Reaktionsprodukt (d. h. ein (chemisches) Produkt oder Produktgemisch), welches eine oder mehrere gegebenenfalls funktionalisierte acylverkappte (acylblockierte) 3-Hydroxybuttersäuren und/oder deren Salze und/oder Ester der allgemeinen Formel (III')

CH₃ - CH(OR³) - CH₂ - C(O)OR⁵ (III')

umfasst,
wobei in der allgemeinen Formel (III')
- der Rest R³ einen Rest CH₃ - C(O) - CH₂ - C(O) - darstellt und
- der Rest R⁵ einen Rest R¹, wobei Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt, oder einen Rest R⁴, wobei der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt, bezeichnet.

Gemäß einer besonderen Ausführungsform der vorliegenden Erfindung kann das Reaktionsprodukt eine oder mehrere acylverkappte (acylblockierte) 3-Hydroxybuttersäuren und/oder deren Salze und/oder Ester der allgemeinen Formel (III)

CH₃ - CH(OR³) - CH₂ - C(O)OR¹ (III)

umfassen,
wobei in der allgemeinen Formel (III) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt und der Rest R³ einen Rest CH₃- C(O) - CH₂ - C(O) - darstellt.

Gemäß einer weiteren besonderen Ausführungsform der vorliegenden Erfindung kann das Reaktionsprodukt eine oder mehrere funktionalisierte acylverkappte (acylblockierte) 3-Hydroxybuttersäuren und/oder deren Salze und/oder Ester der allgemeinen Formel (III")

CH₃ - CH(OR³) - CH₂ - C(O)OR⁴ (III")

umfassen,
wobei in der allgemeinen Formel (III") der Rest R³ einen Rest CH₃- C(O) - CH₂ - C(O) - darstellt und der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt.

Gemäß einer wiederum besonderen Ausführungsform kann das Reaktionsprodukt insbesondere ein Gemisch von mindestens zwei voneinander verschiedenen gegebenenfalls funktionalisierten acylverkappten (acylblockierten) 3-Hydroxybuttersäuren, insbesondere wie zuvor definiert, umfassen.

Gemäß einer weiteren besonderen Ausführungsform kann das Reaktionsprodukt insbesondere ein Gemisch von mindestens drei voneinander verschiedenen gegebenenfalls funktionalisierten acylverkappten (acylblockierten) 3-Hydroxybuttersäuren, insbesondere wie zuvor definiert, umfassen.

Gegenstand der vorliegenden Erfindung ist auch eine gegebenenfalls funktionalisierte acylverkappte (acylblockierte) 3-Hydroxybuttersäure und/oder deren Salz und/oder Ester der allgemeinen Formel (III')

CH₃ - CH(OR³) - CH₂ - C(O)OR⁵ (III')

wobei in der allgemeinen Formel (III')
der Rest R³ einen Rest CH₃ - C(O) - CH₂ - C(O) - darstellt und
der Rest R⁵ einen Rest R¹, wobei der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt, oder einen Rest R⁴, wobei der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt, bezeichnet.

Weiterer Gegenstand der vorliegenden Erfindung ist ebenfalls eine acylverkappte (acylblockierte) 3-Hydroxybuttersäure und/oder deren Salz und/oder Ester, insbesondere wie zuvor beschrieben,
wobei die acylverkappte (acylblockierte) 3-Hydroxybuttersäure und/oder deren Salze und/oder Ester der allgemeinen Formel (III)

   CH₃ - CH(OR³) - CH₂ - C(O)OR¹ (III)
entspricht,
wobei in der allgemeinen Formel (III) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt und der Rest R³ einen Rest CH₃- C(O) - CH₂ -C(O) - darstellt.

Wiederum weiterer Gegenstand der vorliegenden Erfindung ist eine funktionalisierte acylverkappte (acylblockierte) 3-Hydroxybuttersäure und/oder deren Salz und/oder Ester, insbesondere wie zuvor definiert,
wobei die funktionalisierte acylverkappte (acylblockierte) 3-Hydroxybuttersäure und/oder deren Salze und/oder Ester der allgemeinen Formel (III")

   CH₃ - CH(OR³) - CH₂ - C(O)OR⁴ (III")
entspricht,
wobei in der allgemeinen Formel (III") der Rest R³ einen Rest CH₃- C(O) - CH₂ - C(O) - darstellt und der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt.

Weiterer Gegenstand der vorliegenden Erfindung gemäß diesem Erfindungsaspekt ist ein Gemisch, welches mindestens zwei voneinander verschiedene gegebenenfalls funktionalisierte acylverkappte (acylblockierte) 3-Hydroxybuttersäuren und/oder deren Salze und/oder Ester, wie zuvor definiert, umfasst.

Insbesondere ist wiederum weiterer Gegenstand der vorliegenden Erfindung gemäß diesem Erfindungsaspekt ein Gemisch, welches mindestens drei voneinander verschiedene gegebenenfalls funktionalisierte acylverkappte (acylblockierte) 3-Hydroxybuttersäuren und/oder deren Salze und/oder Ester, wie zuvor definiert, umfasst.

Das nach dem erfindungsgemäßen Verfahren erhältliche bzw. erfindungsgemäße Reaktionsprodukt, wie zuvor definiert, und/oder die nach dem erfindungsgemäßen Herstellungsverfahren erhältliche bzw. erfindungsgemäße gegebenenfalls funktionalisierte acylverkappte (acylblockierte) 3-Hydroxybuttersäure, wie zuvor definiert und/oder das nach dem erfindungsgemäßen Herstellungsverfahren erhältliche bzw. erfindungsgemäße Gemisch, wie zuvor definiert, weist gegenüber dem Stand der Technik eine Vielzahl von Vorteilen und Besonderheiten auf:

Wie die Anmelderin überraschend herausgefunden hat, eignet sich das nach dem erfindungsgemäßen Verfahren erhältliche bzw. erfindungsgemäße Reaktionsprodukt, wie zuvor definiert, und/oder die nach dem erfindungsgemäßen Herstellungsverfahren erhältliche bzw. erfindungsgemäße gegebenenfalls funktionalisierte acylverkappte (acylblockierte) 3-Hydroxybuttersäure, wie zuvor definiert, und/oder das nach dem erfindungsgemäßen Herstellungsverfahren erhältliche bzw. erfindungsgemäße Gemisch, wie zuvor definiert, insbesondere als Präkursor bzw. Metabolit von 3-Hydroxybuttersäure bzw. deren Salzen, da dieses bzw. diese einerseits physiologisch, insbesondere im Magen/Darm-Trakt, zu den Ketokörpern 3-Hydroxybuttersäure und 3-Oxobutyrat (= Acetoacetat bzw. Acetacetat), welches physiologisch letztlich zu 3-Hydroxybuttersäure bzw. deren Salzen umgesetzt bzw. reduziert wird, gespalten wird und andererseits gleichzeitig eine gute physiologische Kompatibilität bzw. Verträglichkeit aufweist, insbesondere im Hinblick auf Nichttoxizität und akzeptable organoleptische Eigenschaften. Insbesondere die retardierte Freisetzung des physiologisch wirksamen Stoffes im Magen/Darm-Trakt ist im medizinischen Bereich vorteilhaft, da der Wirkstoff 3-Hydroxybuttersäure so über einen längeren Zeitraum zur Verfügung gestellt werden kann und somit eine Ketose-Therapie ermöglicht wird.

Daher eignen sich das nach dem erfindungsgemäßen Verfahren erhältliche bzw. erfindungsgemäße Reaktionsprodukt, wie zuvor definiert, und/oder die nach dem erfindungsgemäßen Herstellungsverfahren erhältliche bzw. erfindungsgemäße gegebenenfalls funktionalisierte acylverkappte (acylblockierte) 3-Hydroxybuttersäure, wie zuvor definiert, und/oder das nach dem erfindungsgemäßen Herstellungsverfahren erhältliche bzw. erfindungsgemäße Gemisch, wie zuvor definiert, als wirksame Präkursoren oder Metabolite, welche physiologisch einen direkten oder indirekten Zugang zu 3-Hydroxybuttersäure oder deren Salzen sowie zu Acetoacetat (und damit physiologisch wiederum zu 3-Hydroxybuttersäure oder deren Salzen) ermöglichen, insbesondere im physiologischen Stoffwechsel des menschlichen oder tierischen Körpers.

Bei der physiologischen Spaltung im Magen und/oder Darm wird also das nach dem erfindungsgemäßen Verfahren erhältliche bzw. erfindungsgemäße Reaktionsprodukt, wie zuvor definiert, und/oder die nach dem erfindungsgemäßen Herstellungsverfahren erhältliche bzw. erfindungsgemäße gegebenenfalls funktionalisierte acylverkappte (acylblockierte) 3-Hydroxybuttersäure, wie zuvor definiert, und/oder das nach dem erfindungsgemäßen Herstellungsverfahren erhältliche bzw. erfindungsgemäße Gemisch, wie zuvor definiert, in die Ketoverbindungen 3-Hydroxybuttersäure und 3-Oxobutyrat (Acetoacetat bzw. Acetacetat), welches vom Körper weiter zu 3-Hydroxybutyrat reduziert werden kann, gespalten.

Durch die Anwesenheit von sowohl 3-Oxobutyratresten und 3-Hydroxybutyratresten bzw. 3-Hydroxybuttersäure liegt eine unterschiedlich schnelle Verfügbarkeit bzw. Freisetzung des Wirkstoffs 3-Hydroxybuttersäure vor. Das erfindungsgemäße Reaktionsprodukt weist folglich einen intrinsischen, in sich nochmals differenzierten Retard-Effekt auf. Denn insgesamt weist die erfindungsgemäße gegebenenfalls funktionalisierte acylverkappte (acylblockierte) 3-Hydroxybuttersäure bzw. deren Salz oder Ester somit zwei Ketokörper mit unterschiedlich schnellem Abbau auf.

Das erfindungsgemäße Verfahren ermöglicht somit erstmalig die Bereitstellung untoxischer, gegebenenfalls funktionalisierter acylverkappter (acylblockierter) 3-Hydroxybuttersäuren sowie deren Salzen und Estern aus an sich bekannten, kommerziell verfügbaren und vor allem physiologisch unbedenklichen Komponenten bzw. Edukten (Ausgangsverbindungen). Die resultierenden gegebenenfalls funktionalisierten acylverkappten 3-Hydroxybuttersäuren sowie deren Salze und Ester können physiologisch, insbesondere im Magen und/oder im Darm, aufgespalten werden und das Zielmolekül "3-Hydroxybuttersäure" bzw. deren Salze (und daneben auch Acetoacetat, welches physiologisch wiederum weiter zu 3-Hydroxybuttersäure umgesetzt bzw. reduziert werden kann) als Wirkstoff bzw. Wirkkomponente freisetzen bzw. generieren.

Darüber hinaus ist das nach dem erfindungsgemäßen Verfahren erhältliche bzw. erfindungsgemäße Reaktionsprodukt, wie zuvor definiert, und/oder die nach dem erfindungsgemäßen Herstellungsverfahren erhältliche bzw. erfindungsgemäße gegebenenfalls funktionalisierte acylverkappte (acylblockierte) 3-Hydroxybuttersäure, wie zuvor definiert, und/oder das nach dem erfindungsgemäßen Herstellungsverfahren erhältliche bzw. erfindungsgemäße Gemisch, wie zuvor definiert, ohne Weiteres auf synthetischem Wege auch im großtechnischen Maßstab zugänglich bzw. verfügbar, und zwar auch mit der erforderlichen pharmazeutischen bzw. pharmakologischen Qualität.

Zudem kann das nach dem erfindungsgemäßen Verfahren erhältliche bzw. erfindungsgemäße Reaktionsprodukt, wie zuvor definiert, und/oder die gegebenenfalls funktionalisierte acylverkappte (acylblockierte) 3-Hydroxybuttersäure, wie zuvor definiert, und/oder das nach dem erfindungsgemäßen Herstellungsverfahren erhältliche bzw. erfindungsgemäße Gemisch, wie zuvor definiert, in einer enantiomerenreiner bzw. enantiomerenangereicherten Form bereitgestellt werden.

Das nach dem erfindungsgemäßen Verfahren erhältliche bzw. erfindungsgemäße Reaktionsprodukt, wie zuvor definiert, und/oder die nach dem erfindungsgemäßen Herstellungsverfahren erhältliche bzw. erfindungsgemäße gegebenenfalls funktionalisierte acylverkappte (acylblockierte) 3-Hydroxybuttersäure, wie zuvor definiert, und/oder das nach dem erfindungsgemäßen Herstellungsverfahren erhältliche bzw. erfindungsgemäße Gemisch, wie zuvor definiert, stellt somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführend erläutert und im Detail beschrieben.

Wiederum weiterer Gegenstand der vorliegenden Erfindung - gemäß einem **dritten** Aspekt der vorliegenden Erfindung - ist eine pharmazeutische Zusammensetzung, insbesondere ein Arzneimittel oder Medikament, welche(s) ein nach dem erfindungsgemäßen Herstellungsverfahren erhältliches bzw. erfindungsgemäßes Reaktionsprodukt, wie zuvor definiert, und/oder eine nach dem erfindungsgemäßen Herstellungsverfahren erhältliche bzw. erfindungsgemäße gegebenenfalls funktionalisierte acylverkappte (acylblockierte) 3-Hydroxybuttersäure oder deren Salz oder Ester, wie zuvor definiert, und/oder ein nach dem erfindungsgemäßen Herstellungsverfahren erhältliches bzw. erfindungsgemäßes Gemisch, wie zuvor definiert, umfasst.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Wiederum weiterer Gegenstand der vorliegenden Erfindung - gemäß einem **vierten** Aspekt der vorliegenden Erfindung - ist ein nach dem erfindungsgemäßen Herstellungsverfahren erhältliches bzw. erfindungsgemäßes Reaktionsprodukt, wie zuvor definiert, und/oder eine nach dem erfindungsgemäßen Herstellungsverfahren erhältliche bzw. erfindungsgemäße gegebenenfalls funktionalisierte acylverkappte (acylblockierte) 3-Hydroxybuttersäure oder deren Salz oder Ester, wie zuvor definiert, und/oder ein nach dem erfindungsgemäßen Herstellungsverfahren erhältliches bzw. erfindungsgemäßes Gemisch, wie zuvor definiert, zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem **fünften** Aspekt der vorliegenden Erfindung - ist die Verwendung eines Reaktionsprodukts, wie zuvor definiert, und/oder Verwendung mindestens einer acylverkappten (acylblockierten) 3-Hydroxybuttersäure und/oder deren Salz und/oder Ester, wie zuvor definiert, und/oder Verwendung eines Gemischs, wie zuvor definiert, zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem **sechsten** Aspekt der vorliegenden Erfindung - ist die Verwendung eines nach dem erfindungsgemäßen Herstellungsverfahren erhältlichen bzw. erfindungsgemäßen Reaktionsprodukts, wie zuvor definiert, und/oder Verwendung einer nach dem erfindungsgemäßen Herstellungsverfahren erhältlichen bzw. erfindungsgemäßen gegebenenfalls funktionalisierten acylverkappten (acylblockierten) 3-Hydroxybuttersäure oder deren Salz oder Ester, wie zuvor definiert, und/oder Verwendung eines nach dem erfindungsgemäßen Herstellungsverfahren erhältlichen bzw. erfindungsgemäßen Gemischs, wie zuvor definiert, zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

Gleichermaßen weiterer Gegenstand der vorliegenden Erfindung - gemäß einem **siebten** Aspekt der vorliegenden Erfindung - ist ein Nahrungsmittel- und/oder Lebensmittelerzeugnis, welches ein nach dem erfindungsgemäßen Herstellungsverfahren erhältliches bzw. erfindungsgemäßes Reaktionsprodukt, wie zuvor definiert, und/oder eine nach dem erfindungsgemäßen Herstellungsverfahren erhältliche bzw. erfindungsgemäße gegebenenfalls funktionalisierte acylverkappte (acylblockierte) 3-Hydroxybuttersäure oder deren Salz oder Ester, wie zuvor definiert, und/oder ein nach dem erfindungsgemäßen Herstellungsverfahren erhältliches bzw. erfindungsgemäßes Gemisch, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*)*,* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Schließlich ist wiederum weiterer Gegenstand der vorliegenden Erfindung - gemäß einem **achten** Aspekt der vorliegenden Erfindung - die Verwendung eines nach dem erfindungsgemäßen Herstellungsverfahren erhältlichen bzw. erfindungsgemäßen Reaktionsprodukts, wie zuvor definiert, und/oder einer nach dem erfindungsgemäßen Herstellungsverfahren erhältlichen bzw. erfindungsgemäßen gegebenenfalls funktionalisierten acylverkappten (acylblockierten) 3-Hydroxybuttersäure oder deren Salz oder Ester, wie zuvor definiert, und/oder eines nach dem erfindungsgemäßen Herstellungsverfahren erhältlichen bzw. erfindungsgemäßen Gemischs, wie zuvor definiert, in einem Nahrungsmittel- und/oder Lebensmittelerzeugnis.

Gemäß diesem Erfindungsaspekt kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise und Ausgestaltung der vorliegenden Erfindung erläutern sollen.

### AUSFÜHRUNGSBEISPIELE:

### Verwendete Abkürzungen

- 3-BHB = 3-Hydroxybuttersäure bzw. 3-Hydroxybuttersäure-Rest (3-Hydroxybutyrat-Rest)
- 3-BHB-FS = 3-Hydroxybuttersäure (freie Säure)
- 3-BHB-Dimer-Ethylester = Dimer des 3-BHB-Ethylesters
- 3-Acetylaceto-BHB-FS = 3-Acetylacetobuttersäure (freie Säure)
- Acetylaceto-BHB₂-Ethylester = mit Ethylacetoacetat verkapptes Dimer des 3-BHB-Ethylesters

### Herstellungsbeispiele

Das erfindungsgemäße Herstellungsverfahren wird anhand der nachfolgenden Ausführungsbeispiele veranschaulicht. Die diesbezüglichen Reaktionsschemata sind im allgemeinen Beschreibungsteil dargestellt und erläutert.

### Herstellung von 3-Acetylaceto-BHB-Ethylester und Anwendungsversuche

In einem 100-ml-Mehrhalskolben mit Dephlegmator (Partialkondensator) und Destillationsbrücke werden 52 g 3-Oxobuttersäureethylester (Ethylacetoacetat bzw. Acetessigester) und 26 g 3-Hydroxybuttersäureethylester (3-BHB-Ethylester) vorgelegt.

Bei einer Temperatur von 50 °C und unter Vakuum werden 0,8 g immobilisiertes Enzym (CALB-Lipase auf Polymerträger, abgeleitet von *Candida antarctica,* z. B. Novozym^{®} 435) zugegeben. Das Reaktionsgemisch wird unter Rühren für 6 h zur Reaktion gebracht. Das während der Reaktion entstehende Ethanol wird kontinuierlich abdestilliert. Anschließend wird das Enzym abfiltriert und überschüssiger 3-Oxobuttersäureethylester sowie überschüssiger 3-Hydroxybuttersäureethylester unter Vakuum abdestilliert und rezykliert.

Das erhaltene Reaktionsprodukt ist ein 3-Acetylacetobuttersäureethylester (3-Acetylaceto-BHB-Ethylester) und besteht nach analytischer Untersuchung aus folgender Zusammensetzung: > 90 % 3-Acetylaceto-BHB-Ethylester (Reaktionsnebenprodukte: 3-BHB-Dimer-Ethylester < 5 % und Acetylaceto-BHB-Dimer-Ethylester < 5 %).

Die Charakterisierung erfolgt mittels Gaschromatographie (GC) und GC-MS-Analyse (Gaschromatographie mit Massenspektrometrie-Kopplung).

Der Geschmack des 3-Acetylaceto-BHB-Ethylesters ist deutlich weniger unangenehm und bitter als der des reinen 3-BHB-Ethylesters oder gar von reiner 3-Hydroxybuttersäure.

Spaltungsversuche (Spaltversuche) mit 3-Acetylaceto-BHB-Ethylester in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form. Diese Spaltungsversuche belegen, dass acylverkappte (acylblockierte) 3-Hydroxybuttersäure bzw. deren Salze oder Ester, hier konkret 3-Acetylaceto-BHB-Ethylester, effiziente Präkursoren bzw. Metabolite der freien 3-Hydroxybuttersäure bzw. deren Salze und weiterer Ketokörper (hier: Acetoacetat) darstellen, insbesondere im Hinblick auf ihre beabsichtigte Wirkung, wobei diese Verbindungen zudem in physiologisch verträglicher bzw. physiologisch kompatibler Form vorliegen.

### Weitere Herstellung von 3-Acetylaceto-BHB-Ethylester

In einem 100-ml-Mehrhalskolben mit Dephlegmator (Partialkondensator) und Destillationsbrücke werden 30 g 3-Oxobuttersäureethylester (Ethylacetoacetat bzw. Acetessigester) und 15,25 g 3-Hydroxybuttersäureethylester (3-BHB-Ethylester) vorgelegt.

Bei einer Temperatur von 50 °C und unter Vakuum werden 0,46 g immobilisiertem Enzym (CALB-Lipase auf Polymerträger, abgeleitet von *Candida antarctica,* z. B. Novozym^{®} 435) zugegeben. Das Reaktionsgemisch wird unter Rühren für 6 h zur Reaktion gebracht. Das während der Reaktion entstehende Ethanol wird kontinuierlich abdestilliert. Anschließend wird das Enzym abfiltriert und überschüssiger 3-Oxobuttersäureethylester sowie überschüssiger 3-Hydroxybuttersäureethylester unter Vakuum abdestilliert und anschließend rezykliert.

Die Charakterisierung erfolgt mittels Gaschromatographie (GC) und GC-MS-Analyse (Gaschromatographie mit Massenspektrometrie-Kopplung).

Der Umsatz/Zeit-Verlauf wird mittels GC ermittelt. Anhand der mittels GC ermittelten Mengen lässt sich ein Umsatz zum gewünschten Produkt (hier: 3-Acetylaceto-BHB-Ethylester) erkennen. Mit fortschreitender Reaktionszeit findet zudem ein Umsatz des entstandenen 3-BHB-Dimer-Ethylesters mit Ethylacetoacetat zu Acetylaceto-BHB₂-Ethylester (acylverkappter 3-BHB-Dimer-Ethylester) als Nebenprodukt statt (< 1 %).

### Weitere Herstellungsbeispiele

Die Versuche werden mit Natriummethanolat (NaOMe) als Katalysator anstelle des Enzyms und bei Temperaturen zwischen 100 und 120 °C wiederholt. Es werden vergleichbare Ergebnisse erhalten. Aufreinigung und Analyse erfolgen in gleicher Weise.

### Wiederum weitere Herstellungsbeispiele

In einer weiteren Versuchsreihe wird jeweils der Einfluss des Molverhältnisses der Ausgangsverbindungen im Hinblick auf die Bildung von Nebenprodukten (analytisch untersucht anhand der beiden Nebenprodukte "3-BHB-Dimer-Ethylester" und "Acetylaceto-BHB₂-Ethylester") untersucht.

Es zeig sich, dass ein molarer Überschuss an 3-Oxobuttersäureethylester (Ethylacetoacetat bzw. Acetessigester) in Bezug auf das weitere Edukt 3-Hydroxybuttersäureethylester (3-BHB-Ethylester) einer Nebenproduktbildung entgegenwirkt.

In einer ersten Untersuchungsreihe zeigt sich ein Acetessigester / 3-BHB-Ethylester-Molverhältnis im Bereich von 1,5 : 1 bis 9 : 1 besonders effizient in Bezug auf eine Nebenproduktbildung und ist auch noch verfahrensökonomisch. Besonders gute Ergebnisse werden in einer zweiten Untersuchungsreihe für ein Acetessigester / 3-BHB-Ethylester-Molverhältnis im Bereich von 2 : 1 bis 8 : 1 beobachtet.

### Funktionalisierung

In einem 500-ml-Mehrhalskolben mit Dephlegmator (Partialkondensator) und Destillationsbrücke werden 150 g 3-Acetylaceto-BHB-Ethylester, 158 g 1-Decanol und 2,9 g immobilisiertem Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) vorgelegt.

Das Reaktionsgemisch wird unter Rühren bei 70 °C und unter Vakuum (< 500 mbar) für 7 h zur Reaktion gebracht. Das während der Reaktion entstehende Ethanol wird kontinuierlich abdestilliert. Anschließend wird das Enzym abfiltriert und der überschüssige 3-Acetylaceto-BHB-Ethylester bzw. das überschüssige 1-Decanol unter Vakuum abdestilliert. Der erhaltene Rückstand wird für 2 bis 4 h im Hochvakuum gedämpft (Dampftemperatur 160 °C). Es wird reiner 3-Acetylaceto-BHB-Decylester erhalten.

### Weitere Funktionalisierung

In einem 500-ml-Mehrhalskolben mit Dephlegmator (Partialkondensator) und Destillationsbrücke werden 150 g 3-Acetylaceto-BHB-Ethylester, 270 g Oleylalkohol (Reinheit: 85 %) und 4,0 g immobilisiertem Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) vorgelegt.

Das Reaktionsgemisch wird unter Rühren bei 70 °C und unter Vakuum (< 500 mbar) für 7 h zur Reaktion gebracht. Das während der Reaktion entstehende Ethanol wird kontinuierlich abdestilliert. Anschließend wird das Enzym abfiltriert und das Produkt 3-Acetylaceto-BHB-Oleylester im Vakuum durch mehrfache Destillation erhalten. Der erhaltene Rückstand wird bei Bedarf für 2 bis 4 h im Hochvakuum gedämpft (Dampftemperatur 160 °C). Es wird reiner 3-Acetylaceto-BHB-Oleylester erhalten.

### Wiederum weitere Funktionalisierungsversuche

Die vorstehenden enzymkatalysierten Funktionalisierungen werden entsprechend auch mit weiteren Fettalkoholen durchgeführt (nämlich jeweils mit Cetylalkohol, Margarylalkohol, Stearylalkohol, Behenylalkohol, Melissylalkohol, Palmitoleylalkohol und Linoleylalkohol). Es werden die entsprechenden 3-Acetylaceto-BHB-Fettalkoholester jeweils als Reinstoffe erhalten.

### Nochmals weitere Funktionalisierungsversuche

Die vorangehenden Versuche werden wiederholt, jedoch mit Natriummethanolat (NaOMe) als Katalysator (1 Gew.-%) anstelle des Enzyms und bei Temperaturen zwischen 100 und 120 °C. Es werden vergleichbare Ergebnisse erhalten. Aufreinigung und Trennung erfolgen in gleicher Weise.

### Physiologische Anwendungsversuchen in-vitro-Verdauversuche

### Verdauversuche (Spalt- bzw. Spaltungsversuche) von erfindungsgemäßen 3-Acetylaceto-BHB-Estern (d. h. Ethylester und Fettalkoholestern der 3-Acetylacetobuttersäure)

Mittels Spaltungsversuchen wird gezeigt, dass erfindungsgemäß hergestellte 3-Acetylaceto-BHB-Ethylester als auch die funktionalisierten Derivate (d. h. 3-Acetylaceto-BHB-Fettalkoholester), einschließlich der Reaktionsnebenprodukte, wie Dimere etc., im menschlichen gastrointestinalen Trakt gespalten werden können.

Als Testsubstanz werden jeweils aufgereinigte, nach dem erfindungsgemäßen Verfahren erhaltene 3-Acetylaceto-BHB-Ethylester sowie die funktionalisierten Derivate (d.h. 3-Acetylaceto-BHB-Fettalkoholester) eingesetzt.

### Getestete Ester:

- 3-Acetylaceto-BHB-Ethylester
- 3-Acetylaceto-BHB-Decylester
- 3-Acetylaceto-BHB-Oleylester
- 3-Acetylaceto-BHB-Cetylester
- 3-Acetylaceto-BHB-Margarylester
- 3-Acetylaceto-BHB-Stearylester
- 3-Acetylaceto-BHB-Behenylester
- 3-Acetylaceto-BHB-Melissylester
- 3-Acetylaceto-BHB-Palmitoleylester
- 3-Acetylaceto-BHB-Linoleylester

Für die Spaltungsversuche unter köpernahen Bedingungen werden zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®} 40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass die Proben unter FaSSGF-Bedingungen mit Panzytrat^{®} und ohne Panzytrat^{®} hydrolysieren; dies liegt hauptsächlich am niedrigen pH-Wert (pH = 1,6) des Mediums. Bei FaSSIF-Bedingungen findet eine geringere Umsetzung unter Verwendung von Panzytrat^{®} statt.

Die Versuche belegen, dass der 3-Acetylaceto-BHB-Ethylester sowie deren fettalkoholfunktionalisierte Derivate jeweils einen geeigneten physiologischen Präkursor für die Ketokörper 3-Hydroxybuttersäure sowie Acetoacetat (und dadurch letztendlich wieder 3-Hydroxybuttersäure) zur Verwendung in den entsprechenden Ketokörpertherapien darstellen.

### Weitere Verdauversuche (Spaltungsversuche) von erfindungsgemäßen 3-Acetylaceto-BHB-Ethylestern sowie deren funktionalisierte Derivate

### Spaltungsversuche mit Pankreatin

Jeweils 2 g der wie zuvor beschrieben hergestellten 3-Acetoacetat-BHB-Ethylester sowie der wie zuvor beschrieben hergestellten 3-Acetoacetat-BHB-Fettalkoholester werden jeweils in 50 g Wasser gelöst und mit 0,5 g (1 Gew.-%) Pankreatin versetzt. Das Pankreatin wird in Form des kommerziell verfügbaren Produkts Panzytrat^{®} 40.000 von der Fa. Allergan eingesetzt. Das Ganze wird auf einer Heizplatte bei 50 °C gerührt; der Reaktionsverlauf wird mittels kontinuierlicher Erfassung der Säurezahl über die Zeit ermittelt und verfolgt. Die Säurezahl steigt über den Beobachtungszeitraum an (Spaltung des 3-Acetylaceto-BHB-Esters zu der freien 3-Hydroxybuttersäure und Acetoacetat, welches physiologisch wiederum zu 3-BHB bzw. zu 3-Hydroxybutyrat reduziert werden kann). Der Umsatz/Zeit-Verlauf der wässrigen Spaltung der erfindungsgemäßen Ester mittels Pankreatin, einschließlich Zunahme der Säurezahl über die Zeit, belegt die gewünschte Zersetzung des Edukts bzw. Eduktgemischs zu der freien Säure. Dies wird durch entsprechende Analytik bestätigt. Der Versuch belegt, dass sowohl der erfindungsgemäße 3-Acetylaceto-BHB-Ethylester als auch die funktionalisierten Derivate (d.h. 3-Acetylaceto-BHB-Fettalkoholester) geeignete physiologische Präkursoren für 3-Hydroxybuttersäure für die entsprechenden Ketokörpertherapien darstellen. Die Versuche werden jeweils anhand der einzelnen Ester in Reinform wiederholt und verifiziert. Es werden vergleichbare Ergebnisse erhalten, d. h. sowohl die 3-Acetylaceto-BHB-Ethylester als auch die funktionalisierten Derivate werden jeweils durch Pankreatin gespalten.

Die zuvor geschilderten Spaltungsversuche belegen, dass 3-Acetylaceto-BHB-Ethylester, als auch die funktionalisierten Derivate (d.h. 3-Acetylaceto-BHB-Fettalkoholester) effiziente Präkursoren bzw. Metabolite der freien 3-Hydroxybuttersäure bzw. deren Salzen darstellen, insbesondere im Hinblick auf ihre beabsichtigte Wirkung, welche zudem in physiologisch verträglicher bzw. physiologisch kompatibler Form vorliegen.

Die vorliegende Erfindung wird nachfolgend anhand der erfindungsgemäßen Aspekte 1 bis 42 veranschaulicht und offenbart:

### Aspekte 1 bis 42 der vorliegenden Erfindung:

### Aspekt 1:

1. Verfahren zur Herstellung von acylverkappter (acylblockierter) 3-Hydroxybuttersäure (beta-Hydroxybuttersäure, BHB bzw. 3-BHB) oder deren Salz oder Ester,
   wobei mindestens eine Verbindung der allgemeinen Formel (I)

      CH₃ - CH(OH) - CH₂ - C(O)OR¹ (I)
   wobei in der allgemeinen Formel (I) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt,
   mit mindestens einer Verbindung der allgemeinen Formel (II)

      CH₃ - C(O) - CH₂ - C(O)OR² (II)
   wobei in der in der allgemeinen Formel (II) der Rest R² ein C₁-C₄-Alkyl, insbesondere Methyl oder Ethyl, bevorzugt Ethyl, darstellt,
   umgesetzt wird,
   so dass als Reaktionsprodukt mindestens eine acylverkappte (acylblockierte) 3-Hydroxybuttersäure oder deren Salz oder Ester der allgemeinen Formel (III)

      CH₃ - CH(OR³) - CH₂ - C(O)OR¹ (III)
   wobei in der allgemeinen Formel (III) der Rest R¹ die zuvor angegebenen Bedeutung hat und der Rest R³ einen Rest CH₃ - C(O) - CH₂ - C(O) - darstellt,
   erhalten wird.

### Aspekt 2:

2. Verfahren nach Aspekt 1,
wobei die Verbindung der allgemeinen Formel (I) in racemischer Form oder in Form des (R)-Enantiomers eingesetzt wird.

### Aspekt 3:

3. Verfahren nach Aspekt 1 oder Aspekt 2,
wobei in der allgemeinen Formel (I) der Rest R¹ Ethyl darstellt und/oder wobei als Verbindung der allgemeinen Formel (I) 3-Hydroxybuttersäureethylester (Ethyl-3-hydroxybutyrat) der Formel CH₃ - CH(OH) - CH₂ - C(O)OC₂H₅ eingesetzt wird; und/oder
wobei in der allgemeinen Formel (II) der Rest R² Ethyl darstellt und/oder wobei als Verbindung der allgemeinen Formel (II) 3-Oxobuttersäureethylester (Ethyl-3-oxobutyrat) der Formel CH₃ - C(O) - CH₂ - C(O)OC₂H₅ eingesetzt wird.

### Aspekt 4:

4. Verfahren zur Herstellung von acylverkappter (acylblockierter) 3-Hydroxybuttersäure (beta-Hydroxybuttersäure, BHB bzw. 3-BHB) oder deren Salz oder Ester, insbesondere Verfahren nach einem der Aspekte 1 bis 3,
wobei mindestens eine Verbindung der Formel (la)

   CH₃ - CH(OH) - CH₂ - C(O)OC₂H₅ (la)
mit mindestens einer Verbindung der Formel (IIa)

   CH₃ - C(O) - CH₂ - C(O)OC₂H₅ (IIa)
umgesetzt wird,
so dass als Reaktionsprodukt mindestens eine acylverkappte (acylblockierte) 3-Hydroxybuttersäure oder deren Salz oder Ester der Formel (IIIa)

   CH₃ - CH[O - C(O) - CH₂ - C(O) - CH₃] - CH₂ - C(O)OC₂H₅ (IIIa)
erhalten wird.

### Aspekt 5:

5. Verfahren nach einem der vorangehenden Aspekte,
wobei die Umsetzung in Abwesenheit von Lösemitteln und/oder ohne jedwedes Lösemittel durchgeführt wird; und/oder
wobei die Umsetzung in Gegenwart eines Katalysators, insbesondere eines Enzyms und/oder eines metallhaltigen und/oder metallbasierten, sauren oder basischen Katalysators, vorzugsweise in Gegenwart eines Enzyms, durchgeführt wird; insbesondere wobei der Katalysators nach der Umsetzung rezykliert wird.

### Aspekt 6:

6. Verfahren nach einem der vorangehenden Aspekte,
wobei die Umsetzung in Gegenwart eines Enzyms als Katalysator durchgeführt wird;
insbesondere wobei das Enzym ausgewählt wird aus Synthetasen (Ligasen), Katalasen, Esterasen, Lipasen und deren Kombinationen; und/oder
insbesondere wobei das Enzym sich ableitet von *Candida antarctica, Mucor miehei* (*Rhizomucor miehei*), *Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus;* und/oder
insbesondere wobei das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt wird; und/oder
insbesondere wobei das Enzym nach der Umsetzung rezykliert wird; und/oder
insbesondere wobei die Umsetzung in Gegenwart eines Enzyms als Katalysator bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird; und/oder
insbesondere wobei das Enzym in Mengen, bezogen auf die Gesamtmenge der Ausgangsverbindungen (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt wird; und/oder
insbesondere wobei die Umsetzung in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

### Aspekt 7:

7. Verfahren nach einem der vorangehenden Aspekte,
wobei die Umsetzung in Gegenwart eines metallhaltigen und/oder metallbasierten, sauren oder basischen Katalysators durchgeführt wird;
insbesondere wobei der Katalysator ausgewählt ist aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen; und/oder
insbesondere wobei als Katalysator ein Alkali- oder Erdalkalialkoholat eingesetzt wird; und/oder
insbesondere wobei der Katalysator nach der Umsetzung rezykliert wird; und/oder
insbesondere wobei die Umsetzung in Gegenwart eines metallhaltigen und/oder metallbasierten, sauren oder basischen Katalysators bei Temperaturen im Bereich von 20 °C bis 150 °C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt wird; und/oder
insbesondere wobei der Katalysator in Mengen, bezogen auf die Gesamtmenge der Ausgangsverbindungen (I) und (II), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt wird; und/oder
insbesondere wobei die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

### Aspekt 8:

8. Verfahren nach einem der vorangehenden Aspekte,
wobei die Verbindung der allgemeinen Formel (II), bezogen auf die Verbindung der allgemeinen Formel (I), in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird; und/oder
wobei die Verbindung der allgemeinen Formel (II) und die Verbindung der allgemeinen Formel (I) in einem Molverhältnis von Verbindung der allgemeinen Formel (II) / Verbindung der allgemeinen Formel (I) in einem Bereich von 1,1 : 1 bis 10 : 1, bevorzugt in einem Bereich von 1,5 : 1 bis 9 : 1, insbesondere in einem Bereich von 2 : 1 bis 8 : 1, vorzugsweise in einem Bereich von 3 : 1 bis 6 : 1, eingesetzt werden.

### Aspekt 9:

9. Verfahren nach einem der vorangehenden Aspekte,
wobei bei der Umsetzung der mindestens einen Verbindung der allgemeinen Formel (I) mit mindestens einer Verbindung der allgemeinen Formel (II) gleichzeitig eine Verbindung gemäß der allgemeinen Formel (IV)

   R² - OH (IV)
wobei der Rest R² die zuvor angegebene Bedeutung hat, gebildet wird;
insbesondere wobei die Verbindung gemäß der allgemeinen Formel (IV) der Umsetzung insbesondere kontinuierlich entzogen wird, insbesondere mittels vorzugsweise kontinuierlicher destillativer Entfernung.

### Aspekt 10:

10. Verfahren nach einem der vorangehenden Aspekte,
wobei sich der Umsetzung der mindestens einen Verbindung der allgemeinen Formel (I) mit mindestens einer Verbindung der allgemeinen Formel (II) eine Aufreinigung anschließt, insbesondere mittels Destillation und/oder Chromatographie, vorzugsweise mittels Destillation;
insbesondere wobei gegebenenfalls noch vorhandene Edukte und Reaktionsnebenprodukte, insbesondere Verbindungen gemäß der allgemeinen Formel (IV), abgetrennt werden, insbesondere abdestilliert werden; und/oder
insbesondere wobei gegebenenfalls noch vorhandene Edukte, insbesondere Edukte der allgemeinen Formeln (I) und (II), nach deren Abtrennung rezykliert werden.

### Aspekt 11:

11. Verfahren nach einem der vorangehenden Aspekte,
wobei nach erfolgter Umsetzung das Reaktionsprodukt (III) an dessen Rest R¹ zumindest teilweise, vorzugsweise vollständig, funktionalisiert wird, vorzugsweise durch Veresterung oder Umesterung; und/oder
wobei sich der Umsetzung eine teilweise, insbesondere vollständige, Funktionalisierung des Reaktionsprodukts (III) an dessen Rest R¹ anschließt, vorzugsweise durch Veresterung oder Umesterung.

### Aspekt 12:

12. Verfahren nach Aspekt 11,
wobei das Reaktionsprodukt (III) mit mindestens einem Fettalkohol (V), vorzugsweise ausgewählt aus C₆-C₃₀-Fettalkoholen, bevorzugt C₁₀-C₃₀-Fettalkoholen, insbesondere C₁₀-C₂₄-Fettalkoholen, funktionalisiert wird.

### Aspekt 13:

13. Verfahren nach Aspekt 11 oder Aspekt 12,
wobei der Fettalkohol (V) der allgemeinen Formel (V')

   R⁴-OH (V')
entspricht, wobei der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt, insbesondere wobei die Hydroxylfunktion (OH-Funktion) primär und/oder endständig ist.

### Aspekt 14:

14. Verfahren nach Aspekt 13,
wobei der Rest R⁴ einen linearen, gesättigten oder ein- oder mehrfach ungesättigten aliphatischen C₁₀-C₂₄-Alkylrest darstellt, insbesondere wobei die Hydroxylfunktion (OH-Funktion) primär und/oder endständig ist; und/oder
wobei der Rest R⁴ einen 1-Decanylrest, einen 1-Dodecanylrest (Laurylrest), einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen cis-9-Hexadecen-1-ylrest (Palmitoleylrest), einen cis-9-Octadecen-1-ylrest (Oleylrest), einen trans-9-Octadecen-1-ylrest (Elaidylrest), einen cis-11-Octadecen-1-ylrest, einen cis,cis-9,12-Octa-decadien-1-ylrest (Linoleylrest) oder einen 6,9,12-Octadecatrien-1-ylrest (γ-Linolenylrest), bevorzugt einen cis-9-Octadecen-1-ylrest (Oleylrest), darstellt.

### Aspekt 15:

15. Verfahren nach einem der Aspekte 12 bis 14,
wobei der Fettalkohol (V) ausgewählt ist aus linearen oder verzweigten, gesättigten oder ein- oder mehrfach ungesättigten aliphatischen C₆-C₃₀-Fettalkoholen, vorzugsweise C₁₀-C₃₀-Fettalkoholen, insbesondere C₁₀-C₂₄-Fettalkoholen, bevorzugt mit primärer und/oder endständiger Hydroxylfunktion (OH-Funktion); und/oder
wobei der Fettalkohol (V) ausgewählt ist aus linearen, gesättigten oder ein- oder mehrfach ungesättigten, aliphatischen einwertigen und bevorzugt primären C₆-C₃₀-Fettalkoholen, vorzugweise linearen, gesättigten oder ein-oder mehrfach ungesättigten, aliphatischen einwertigen und bevorzugt primären C₁₀-C₃₀-Fettalkoholen insbesondere linearen, gesättigten oder ein-oder mehrfach ungesättigten, aliphatischen einwertigen und bevorzugt primären C₁₀-C₂₄-Fettalkoholen; und/oder
wobei der Fettalkohol (V) ausgewählt ist aus der Gruppe von 1-Decanol, 1-Dodecanol (Laurylalkohol), 1-Tetradecanol (Myristylalkohol), 1-Hexadecanol (Cetylalkohol), 1-Heptadecanol (Margarylalkohol), 1-Octadecanol (Stearylalkohol), 1-Eicosanol (Arachidylalkohol), 1-Docosanol (Behenylalkohol), 1-Tetracosanol (Ligocerylalkohol), 1-Hexacosanol (Cerylalkohol), 1-Octacosanol (Montanylalkohol), 1-Tricontanol (Melissylalkohol), cis-9-Hexa-decen-1-ol (Palmitoleylalkohol), cis-9-Octadecen-1-ol (Oleylalkohol), *trans-9-*Octadecen-1-ol (Elaidylalkohol), cis-11-Octadecen-1-ol, cis,cis-9,12-Octa-decadien-1-ol (Linoleylalkohol), 6,9,12-Octadecatrien-1-ol (γ-Linolenylalkohol), und deren Mischungen, bevorzugt cis-9-Octadecen-1-ol (Oleylalkohol).

### Aspekt 16:

16. Verfahren nach einem der Aspekte 11 bis 15,
wobei die Funktionalisierung in Abwesenheit von Lösemitteln und/oder ohne jedwedes Lösemittel durchgeführt wird; und/oder
wobei die Funktionalisierung in Gegenwart eines Katalysators, insbesondere eines Enzyms und/oder eines metallhaltigen und/oder metallbasierten, sauren oder basischen Katalysators, vorzugsweise in Gegenwart eines Enzyms, durchgeführt wird; insbesondere wobei der Katalysators nach der Funktionalisierung rezykliert wird.

### Aspekt 17:

17. Verfahren nach einem der Aspekte 11 bis 16,
wobei die Funktionalisierung in Gegenwart eines Enzyms als Katalysator durchgeführt wird;
insbesondere wobei das Enzym ausgewählt wird aus Synthetasen (Ligasen), Katalasen, Esterasen, Lipasen und deren Kombinationen; und/oder
insbesondere wobei das Enzym sich ableitet von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei (Rhizomucor miehei)* und *Thermomyces lanuginosus;* und/oder
insbesondere wobei das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt wird; und/oder
insbesondere wobei das Enzym nach der Funktionalisierung rezykliert wird; und/oder
insbesondere wobei die Funktionalisierung in Gegenwart eines Enzyms als Katalysator bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird; und/oder
insbesondere wobei das Enzym in Mengen, bezogen auf die Gesamtmenge der Verbindungen (III) und (V), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt wird; und/oder
insbesondere wobei die Funktionalisierung in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

### Aspekt 18:

18. Verfahren nach einem der Aspekte 11 bis 17,
wobei die Funktionalisierung in Gegenwart eines metallhaltigen und/oder metallbasierten, sauren oder basischen Katalysators durchgeführt wird;
insbesondere wobei der Katalysator ausgewählt ist aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen; und/oder
insbesondere wobei als Katalysator ein Alkali- oder Erdalkalialkoholat eingesetzt wird; und/oder
insbesondere wobei der Katalysator nach der Funktionalisierung rezykliert wird; und/oder
insbesondere wobei die Funktionalisierung in Gegenwart eines metallhaltigen und/oder metallbasierten, sauren oder basischen Katalysators bei Temperaturen im Bereich von 20 °C bis 150 °C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt wird; und/oder
insbesondere wobei der Katalysator in Mengen, bezogen auf die Gesamtmenge der Verbindungen (III) und (V), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt wird; und/oder
insbesondere wobei die Funktionalisierung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

### Aspekt 19:

19. Verfahren nach einem der Aspekte 11 bis 18,
wobei bei der Funktionalisierung gleichzeitig eine Verbindung gemäß der allgemeinen Formel (VI)

   R¹ - OH (VI)
gebildet wird, wobei in der allgemeinen Formel (VI) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt;
insbesondere wobei die Verbindung gemäß der allgemeinen Formel (VI) der Funktionalisierung entzogen wird, insbesondere kontinuierlich entzogen wird, insbesondere mittels vorzugsweise kontinuierlicher destillativer Entfernung.

### Aspekt 20:

20. Verfahren nach einem der vorangehenden Aspekte,
wobei als Reaktionsprodukt ein oder mehrere gegebenenfalls funktionalisierte acylverkappte (acylblockierte) 3-Hydroxybuttersäuren und/oder deren Salze und/oder Ester der allgemeinen Formel (III')

   CH₃ - CH(OR³) - CH₂ - C(O)OR⁵ (III')
gebildet werden,
wobei in der allgemeinen Formel (III') der Rest R³ einen Rest CH₃- C(O) - CH₂ - C(O) - darstellt und der Rest R⁵ einen Rest R¹, wie zuvor definiert, und/oder einen Rest R⁴, wie zuvor definiert, darstellt.

### Aspekt 21:

21. Verfahren nach einem der vorangehenden Aspekte,
wobei als Reaktionsprodukt ein oder mehrere acylverkappte (acylblockierte) 3-Hydroxybuttersäuren und/oder deren Salze und/oder Ester der allgemeinen Formel (III)

   CH₃ - CH(OR³) - CH₂ - C(O)OR¹ (III)
gebildet werden,
wobei in der allgemeinen Formel (III) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt und der Rest R³ einen Rest CH₃ - C(O) - CH₂ - C(O) - darstellt.

### Aspekt 22:

22. Verfahren nach einem der vorangehenden Aspekte,
wobei als Reaktionsprodukt ein oder mehrere funktionalisierte acylverkappte (acylblockierte) 3-Hydroxybuttersäuren und/oder deren Salze und/oder Ester der allgemeinen Formel (III")

   CH₃ - CH(OR³) - CH₂ - C(O)OR⁴ (III")
gebildet werden,
wobei in der allgemeinen Formel (III") der Rest R³ einen Rest CH₃- C(O) - CH₂ - C(O) - darstellt und der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt.

### Aspekt 23:

23. Reaktionsprodukt, erhältlich gemäß dem Verfahren nach einem der vorangehenden Aspekte.

### Aspekt 24:

24. Reaktionsprodukt, insbesondere (chemisches) Produkt oder Produktgemisch, insbesondere Reaktionsprodukt nach Aspekt 23,
wobei das Reaktionsprodukt eine oder mehrere gegebenenfalls funktionalisierte acylverkappte (acylblockierte) 3-Hydroxybuttersäuren und/oder deren Salze und/oder Ester der allgemeinen Formel (III')

   CH₃ - CH(OR³) - CH₂ - C(O)OR⁵ (III')
umfasst,
wobei in der allgemeinen Formel (III')
   - der Rest R³ einen Rest CH₃ - C(O) - CH₂ - C(O) - darstellt und
   - der Rest R⁵ einen Rest R¹, wobei Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt, oder einen Rest R⁴, wobei der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt, bezeichnet.

### Aspekt 25:

25. Reaktionsprodukt nach Aspekt 23 oder 24,
wobei das Reaktionsprodukt eine oder mehrere acylverkappte (acylblockierte) 3-Hydroxybuttersäuren und/oder deren Salze und/oder Ester der allgemeinen Formel (III)

   CH₃ - CH(OR³) - CH₂ - C(O)OR¹ (III)
umfasst,
wobei in der allgemeinen Formel (III) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt und der Rest R³ einen Rest CH₃ - C(O) - CH₂ -C(O) - darstellt.

### Aspekt 26:

26. Reaktionsprodukt nach Aspekt 23 oder 24,
wobei das Reaktionsprodukt eine oder mehrere funktionalisierte acylverkappte (acylblockierte) 3-Hydroxybuttersäuren und/oder deren Salze und/oder Ester der allgemeinen Formel (III")

   CH₃ - CH(OR³) - CH₂ - C(O)OR⁴ (III")
umfasst,
wobei in der allgemeinen Formel (III") der Rest R³ einen Rest CH₃ - C(O) - CH₂ - C(O) - darstellt und der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt.

### Aspekt 27:

27. Reaktionsprodukt nach einem der vorangehenden Aspekte,
wobei das Reaktionsprodukt ein Gemisch von mindestens zwei voneinander verschiedenen gegebenenfalls funktionalisierten acylverkappten (acylblockierten) 3-Hydroxybuttersäuren, insbesondere wie zuvor definiert, umfasst.

### Aspekt 28:

28. Reaktionsprodukt nach einem der vorangehenden Aspekte,
wobei das Reaktionsprodukt ein Gemisch von mindestens drei voneinander verschiedenen gegebenenfalls funktionalisierten acylverkappten (acylblockierten) 3-Hydroxybuttersäuren, insbesondere wie zuvor definiert, umfasst.

### Aspekt 29:

29. Gegebenenfalls funktionalisierte acylverkappte (acylblockierte) 3-Hydroxybuttersäure und/oder deren Salz und/oder Ester der allgemeinen Formel (III')

CH₃ - CH(OR³) - CH₂ - C(O)OR⁵ (III')

wobei in der allgemeinen Formel (III')
der Rest R³ einen Rest CH₃ - C(O) - CH₂ - C(O) - darstellt und
der Rest R⁵ einen Rest R¹, wobei der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt, oder einen Rest R⁴, wobei der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt, bezeichnet.

### Aspekt 30:

30. Acylverkappte (acylblockierte) 3-Hydroxybuttersäure und/oder deren Salz und/oder Ester, insbesondere nach Aspekt 29,
wobei die acylverkappte (acylblockierte) 3-Hydroxybuttersäure und/oder deren Salze und/oder Ester der allgemeinen Formel (III)

   CH₃- CH(OR³) - CH₂ - C(O)OR¹ (III)
entspricht,
wobei in der allgemeinen Formel (III) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt und der Rest R³ einen Rest CH₃ - C(O) - CH₂ - C(O) - darstellt.

### Aspekt 31:

31. Funktionalisierte acylverkappte (acylblockierte) 3-Hydroxybuttersäure und/oder deren Salz und/oder Ester, insbesondere nach Aspekt 29,
wobei die funktionalisierte acylverkappte (acylblockierte) 3-Hydroxybuttersäure und/oder deren Salze und/oder Ester der allgemeinen Formel (III")

   CH₃ - CH(OR³) - CH₂ - C(O)OR⁴ (III")
entspricht,
wobei in der allgemeinen Formel (III") der Rest R³ einen Rest CH₃- C(O) - CH₂ - C(O) - darstellt und der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt.

### Aspekt 32:

32. Gemisch, umfassend mindestens zwei voneinander verschiedene gegebenenfalls funktionalisierte acylverkappte (acylblockierte) 3-Hydroxybuttersäuren und/oder deren Salze und/oder Ester, wie zuvor definiert.

### Aspekt 33:

33. Gemisch, umfassend mindestens drei voneinander verschiedene gegebenenfalls funktionalisierte acylverkappte (acylblockierte) 3-Hydroxybuttersäuren und/oder deren Salze und/oder Ester, wie zuvor definiert.

### Aspekt 34:

34. Pharmazeutische Zusammensetzung, insbesondere Arzneimittel oder Medikament, umfassend ein Reaktionsprodukt gemäß einem der Aspekte 23 bis 28 und/oder mindestens eine acylverkappte (acylblockierte) 3-Hydroxybuttersäure und/oder deren Salz und/oder Ester gemäß einem der Aspekte 29 bis 31 und/oder ein Gemisch gemäß Aspekt 32 oder Aspekt 33.

### Aspekt 35:

35. Pharmazeutische Zusammensetzung nach Aspekt 34 zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

### Aspekt 36:

36. Reaktionsprodukt gemäß einem der Aspekte 23 bis 28 und/oder mindestens eine acylverkappte (acylblockierte) 3-Hydroxybuttersäure und/oder deren Salz und/oder Ester gemäß einem der Aspekte 29 bis 31 und/oder ein Gemisch gemäß Aspekt 32 oder Aspekt 33 zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

### Aspekt 37:

37. Verwendung eines Reaktionsprodukts gemäß einem der Aspekte 23 bis 28 und/oder Verwendung mindestens einer acylverkappten (acylblockierten) 3-Hydroxybuttersäure und/oder deren Salz und/oder Ester gemäß einem der Aspekte 29 bis 31 und/oder Verwendung eines Gemischs gemäß Aspekt 32 oder Aspekt 33 zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

### Aspekt 38:

38. Verwendung eines Reaktionsprodukts gemäß einem der Aspekte 23 bis 28 und/oder Verwendung mindestens einer acylverkappten (acylblockierte) 3-Hydroxybuttersäure und/oder deren Salz und/oder Ester gemäß einem der Aspekte 29 bis 31 und/oder Verwendung eines Gemischs gemäß Aspekt 32 oder Aspekt 33 zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

### Aspekt 39:

39. Nahrungsmittel- und/oder Lebensmittelerzeugnis, umfassend ein Reaktionsprodukt gemäß einem der Aspekte 23 bis 28 und/oder mindestens eine gegebenenfalls (acylblockierte) 3-Hydroxybuttersäure und/oder deren Salz und/oder Ester gemäß einem der Aspekte 29 bis 31 und/oder ein Gemisch gemäß Aspekt 32 oder Aspekt 33.

### Aspekt 40:

40. Nahrungsmittel- und/oder Lebensmittelerzeugnis nach Aspekt 39, wobei das Nahrungsmittel- und/oder Lebensmittelerzeugnis ein Nahrungsergänzungsmittel, ein funktionelles Lebensmittel *(Functional Food),* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement ist.

### Aspekt 41:

41. Verwendung eines Reaktionsprodukts gemäß einem der Aspekte 23 bis 28 und/oder mindestens einer acylverkappten (acylblockierte) 3-Hydroxybuttersäure und/oder deren Salz und/oder Ester gemäß einem der Aspekte 29 bis 31 und/oder eines Gemischs gemäß Aspekt 32 oder Aspekt 33 in einem Nahrungsmittel- und/oder Lebensmittelerzeugnis.

### Aspekt 42:

42. Verwendung nach Aspekt 41, wobei das Nahrungsmittel- und/oder Lebensmittelerzeugnis ein Nahrungsergänzungsmittel, ein funktionelles Lebensmittel *(Functional Food),* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement ist.

## Patentansprüche

1. Funktionalisierte acylverkappte (acylblockierte) 3-Hydroxybuttersäure und/oder deren Salz und/oder Ester,
wobei die funktionalisierte acylverkappte (acylblockierte) 3-Hydroxybuttersäure und/oder deren Salz und/oder Ester der allgemeinen Formel (III")
CH₃ - CH(OR³) - CH₂ - C(O)OR⁴ (III")
entspricht,
wobei in der allgemeinen Formel (III") der Rest R³ einen Rest CH₃- C(O) - CH₂ - C(O) - darstellt und der Rest R⁴ einen linearen oder verzweigten, gesättigten oder ein- oder mehrfach ungesättigten aliphatischen C₆-C₃₀-Alkylrest, vorzugsweise C₁₀-C₃₀-Alkylrest, bevorzugt C₁₀-C₂₄-Alkylrest, darstellt.

2. Pharmazeutische Zusammensetzung, insbesondere Arzneimittel oder Medikament, umfassend mindestens eine funktionalisierte acylverkappte (acylblockierte) 3-Hydroxybuttersäure und/oder deren Salz und/oder Ester gemäß Anspruch 1.

3. Pharmazeutische Zusammensetzung nach Anspruch 2 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

4. Nahrungsmittel- und/oder Lebensmittelerzeugnis, umfassend mindestens eine funktionalisierte acylverkappte (acylblockierte) 3-Hydroxybuttersäure und/oder deren Salz und/oder Ester gemäß Anspruch 1.
